# EUROPEAN PATENT SPECIFICATION

(11) **EP 1 384 472 B1**
(45) Date of publication and mention of the grant of the patent: **22.02.2006**
(21) Application number: 01996373.5
(22) Date of filing: 14.11.2001
(51) Int. Cl.: A61K 31/01, A61P 39/06, C09B 61/00, B01D 11/02

(54) **METHOD FOR PRODUCING AN ORGANIC SOLVENT-FREE LYCOPENE CONCENTRATE, THE RESULTING CONCENTRATE AND COMPOSITION COMPRISING SAID CONCENTRATE**
METHODE ZUR HERSTELLUNG LYCOPEN-KONZENTRATS FREI VON ORGANISCHEN LÖSUNGSMITTELN, DAS RESULTIERENDE KONZENTRAT UND DIESES KONZENTRAT ENTHALTENDE ZUSAMMENSETZUNGEN
PROCEDE D'OBTENTION D'UN CONCENTRE DE LYCOPENE SANS SOLVANTS ORGANIQUES, CONCENTRE AINSI OBTENU ET COMPOSITION CONTENANT LEDIT CONCENTRE

(30) Priority: 15.11.2000 ES 200002739
(43) Date of publication of application: 28.01.2004
(73) Proprietor: Universidad de Extremadura, 08720 Badajoz (ES)
(72) Inventor: SABIO REY, E., Escuela Ingenierias Industriales, 06071 Badajoz (ES); RAMIRO GONZALEZ, A., Escuela Ingenierias Industr., 06071 Badajoz (ES); GONZALEZ GONZALEZ, J.F., Esc. Ingenierias Industr., 06071 Badajoz (ES); CASTRO GOMEZ, F.J., Escuela Ingenierias Industr., 06071 Badajoz (ES); BERNALTE GARCIA, M.J., Esc. Ingenierias Agrarias, 06071 Badajoz (ES); MORENO DE ESPINOSA TENA, V., Inst. Tecnologico, Carretera de Cáceres s/n, 06071 Badajoz (ES); HERNANDEZ MENDEZ, T., Inst. Tecnologico Agroalimen, Carretera de Cáceres s/n, 06071 Badajoz (ES); COELHO, José A., Inst. Superior de Ing. de Lisboa, 1949-014 Lisboa (PT); F. PALAVRA, Antonio, Dept. de Ingenieria Quimica, 01049 001 Lisboa (PT); LOZANO RUIZ, M., Inst. Tecnologico Agroalimentario, Carretera de Caceres s/n, 06071 Badajoz (ES)
(74) Representative: Carpintero Lopez, Francisco
(86) International application number: PCT/ES2001/000433
(87) International publication number: WO 2002/040003

(56) References cited:
- WO-A-01/79355
- FR-A1- 2 792 831
- BAYSAL T. ET AL.: 'Supercritical CO2 extraction of beta-carotene and lycopene from tomato paste waste' J. AGRIC. FOOD CHEM. vol. 48, no. 11, 14 October 2000, pages 5507 - 5511, ISSN 0021-8561, XP001095545
- CADONI E. ET AL.: 'Supercritical CO2 extraction of lycopene and beta-carotene from ripe tomatoes' DYES PIGM. vol. 44, no. 1, 1999, pages 27 - 32, ISSN 0143-7208, XP004362773

## Description

### FIELD OF THE INVENTION

The invention concerns a lycopene concentrate free of organic solvents, the procedure used to obtain this and the compositions of this concentrate. These concentrates and compositions can be used to produce food products, cosmetics, pharmaceutical products or nutraceutical agents.

### BACKGROUND OF THE INVENTION

Lycopene is a carotenoid responsible for the red colour of a large number of fruit and vegetables. This compound has some remarkable properties as a colorant and, although it has a similar composition to β-carotene, it is a much more effective pigment. In this sense, it must be mentioned that lycopene covers a wider range of colours ranging from light yellow, passing through orange, to an intense red colour. Moreover, its colour intensity is greater and in the yellow-orange range this is 6 to 8 times stronger than that of β-carotene. It is authorised for use as a food colorant and its code in the European Union is E-160d.

Although use of lycopene as a colorant is interesting, undoubtedly, its antioxidant potential is its most exceptional property. In the organism, oxidations occur on a cellular level due to the presence of free radicals and especially of the oxygen singlet. These undesirable reactions are very dangerous since, like other reactions involving radicals, they are autocatalytic, i.e. they self-propagate via a chain reaction process. As a result, irreversible damage can be produced in essential cellular components (membrane lipids, nucleic acids etc.), in a process known as oxidative stress, which is associated with cell ageing, degenerative diseases, occluded arteries and the appearance of different types of cancer [Castro (1999)] (see the REFERENCES section).

Lycopene has a strong antioxidant potential [Burton (1989); Diplock (1991)] that makes it an excellent deactivator of the oxygen singlet and free radicals [Di Mascio et al., (1989), (1991)]. This natural pigment acts as an antioxidant agent giving up electrons to the free radicals, deactivating them. This antioxidant potential confers them an anticarcerigenous activity and a capacity to help prevent cardiovascular diseases. Studies of Giovannucci (1998) and Giovannucci et al. (1995), indicate that consumption of tomatoes, tomato sauce and pizza is directly associated with a reduced risk of developing different types of cancer, such as cancer of the digestive system and prostate cancer.

Cardiovascular diseases are among the main causes of mortality in western countries. Initially, elevated plasma cholesterol levels were considered to be one of the main risk factors for these conditions. Later, oxidation of cholesterol by the action of free radicals was considered to be the key stage in atherogenesis. It has been demonstrated that the incidence of cardiovascular diseases is strongly associated with plasma levels of cartooned, and lycopene is particularly effective at clearing away peroxide radicals under physiological conditions and preventing the oxidation of low molecular weight lipoproteins (LDL) to their atherogenic form.

Lycopene, because of its special properties, is a true nutraceutical agent. A nutraceutical agent is defined as "a food product, or part of one, that provides medical or health benefits, including the prevention and treatment of diseases" [De Felice, (1991)].

The production of most concentrates or products rich in lycopene, or in other cartooned, currently available on the market requires the use of organic solvents [WO 96/13178, EP 671 461 A1] . Since organic solvents present a greater or lesser degree of toxicity, both for the operators who make the products and also for the consumers, it is recommendable not to use organic solvents to make nutraceutical products or those for pharmacological use since their total elimination cannot be guaranteed. Neither does the use of synthetic lycopene ensure the absence of organic solvents since these are used during their synthesis.

There is, therefore, the need to elaborate a procedure via which a lycopene concentrate free of organic solvents can be obtained.

The invention provides a solution to this necessity that is based on the use of a fluid in supercritical conditions to extract the lycopene present in a raw material that contains it. The extraction of lycopene using a supercritical fluid presents the advantage that, after finishing the extraction, this fluid can be totally eliminated without leaving any trace in the concentrate.

This extraction of lycopene using supercritical CO₂ extraction is known in the state of the art. Baysal et al. ("Supercritical CO₂ extraction of beta-carotene and lycopene from tomato paste waste", *J*. *Agric. Food Chem.* Vol. 48, No. 11, 2000) discloses a procedure for obtaining a lycopene concentrate consisting of drying of a tomato paste (with a humidity of about 6%), extracting of lycopene with liquid CO₂, and further separation of lycopene after depressurization. Also, Cadoni et al. ("Supercritical CO2 extraction of lycopene and beta-carotene from ripe tomatoes", Dyes and Pigments 44 (2000) 27-32) discloses a procedure for the isolation of lycopene consisting of drying of tomato skins and dried seeds, extracting of lycopene with liquid CO₂, and recovering of lycopene in a vial connected to a restrictor (depressurization step). In these studies, the process conditions during the extraction of lycopene have been optimized with respect to pressure, temperature, cosolvent addition, etc.

The present inventors have unexpectedly found out that the selection of the conditions for the final depressurization step results in a superior lycopene content

Consequently, an object of this invention is a lycopene concentrate free of organic solvents, from hereon referred to as lycopene concentrate FOS.

An additional objective of this invention is a procedure to obtain this lycopene concentrate FOS.

Another additional objective of this invention is the composition of this lycopene concentrate FOS together with an appropriate diluent and, optionally, with one or more acceptable additives.

### DETAILLED DESCRIPTION OF THE INVENTION

The invention provides a procedure to obtain a lycopene concentrate FOS, via supercritical extraction of lycopene contained in a lycopene source, from hereon referred to as the procedure of the invention, that includes:
a) placing a source of lycopene in contact with a supercritical fluid, under conditions that permit the solubilisation of lycopene in this supercritical fluid; and
b) separating the lycopene concentrate free of organic solvents by depressurising the supercritical fluid loaded with the lycopene obtained in stage a) characterised in that the supercritical fluid loaded with lycopene is depressurised to a pressure equal to or higher than 10 MPa and to a temperature between 40°C and 60°C;

As a source of lycopene (raw material) any product that contains lycopene can be used, for example, tomatoes or watermelons. In one specific application, this lycopene source corresponds to the industrial wastes generated in the tomato processing industry. This by-product, either composed of the tomato skins or the tomato skins and seeds, when appropriately treated, is an excellent source of lycopene.

As an example, the concentration of lycopene in a lycopene concentrate FOS obtained from the industrial wastes of tomato processing, comprised of tomato skins and seeds, by the procedure of the invention, can reach up to, approximately, 10,000 ppm, i.e. 10 g of lycopene per kg of concentrate, depending on the variety of tomato and the conditions used in the procedure of the invention, whereas fresh tomato usually contains between 20 and 100 mg per kg of tomato (20-100 ppm) (although some varieties that have been developed recently have up to 250 mg per kg of tomato). However, when the source of lycopene is comprised totally of tomato skins, the concentration of lycopene that can be obtained in the lycopene concentrate F_{OS} is even higher, around 37g per kg of concentrate (37,000 ppm), since the seeds do not contain lycopene and have a high lipid contents, thus only exert a dilution effect.

The use of tomato skins as a lycopene source has additional advantages, apart from producing an extract with a higher lycopene contents, which include the following:
i) the extraction of lycopene is much quicker (several tests carried out by the inventors have demonstrating that using tomato skins, 70% of the lycopene can be extracted in one hour, performing the supercritical extraction at a pressure of 30 MPa and a temperature of 80°C, whereas when the lycopene source is comprised of tomato skins and seeds, operating under the same conditions, and for the same length of time, only 25% was extracted; and
ii) the fat from the seeds is polyunsaturated (with a linoleic content above 50%) and, is, therefore, easily oxidised.

On the other hand, the use of these by-products from the tomato processing industry provides a solution to the environmental problem associated with industrial tomato wastes, which have almost no commercial value and usually, therefore, accumulate in an uncontrolled manner, rapidly initiating a fermentation process, with the associated environmental and health problems that this can entail.

The source of lycopene, before coming into contact with the supercritical fluid, is dried until it reaches a suitable degree of humidity. In one specific application, the lycopene source is dried until it obtains a humidity between 1 and 10%, since higher humidities can hinder the supercritical extraction process whereas humidities lower than 1% imply the use of very extreme drying conditions or the use of very expensive drying equipment. If the drying process cannot be carried out immediately, it is recommendable to store the lycopene source in cool conditions to avoid degradation of the lycopene.

The lycopene source, with the appropriate degree of humidity, is ground or milled to obtain a suitable particle size to facilitate extraction with the supercritical fluid. In one specific application, the lycopene source is milled to obtain a particle size between 0.3 and 1.5 mm. In general, reduction of the particle size increases the yield and the extraction rate. However, very small particle sizes can cause technical problems, for example, a potentially important pressure loss. A pressure loss implies a reduced density of the supercritical fluid and, therefore, a reduced extraction capacity.

The supercritical fluid is a fluid that is found at temperatures and pressures above its corresponding critical values, it has properties of both states, liquid and gas, such as high density and diffusivity. For application of the procedure of the invention, any supercritical fluid can be used that is innocuous and capable of extracting lycopene, for example, carbon dioxide, ethylene, ethane, chlorotrifluoromethane, propylene etc. In one specific application, the supercritical fluid is carbon dioxide, a product capable of extracting the apolar components present in the lycopene source, for reasons of low cost, innocuous nature and other advantages [Rizvi et al., (1986)].

The process of supercritical extraction is carried out in conventional supercritical extraction equipment that consists of a supercritical fluid tank, a compressor, an extractor, one or more separators, a thermostatisation system and some pressure reduction valves.

To carry out the supercritical extraction, the lycopene source, adequately treated, is introduced into the extractor and a supercritical fluid is passed through the bed of solid starting material, under pressure and temperature conditions which permit the solubilisation of the lycopene in the supercritical fluid. As the supercritical fluid crosses the bed of starting material, the supercritical fluid extracts the soluble components and then moves on to the separators, where the desired product is obtained.

In general, in the supercritical extraction stage itself [(stage a)], the pressure and temperature conditions chosen are such that they permit an adequate solubilisation of the lycopene. Therefore, in one specific application, the supercritical extraction stage is carried out under high pressure, preferentially, equal to or greater than 30 MPa, usually between 30 and 70 MPa. The temperature in the extraction stage can vary within a wide interval since the solubility is a function of the pressure-temperature combination. In one specific application, the temperature in the supercritical extraction stage is between 50°C and 80°C, since temperatures above 80°C can induce degradation of the extracted material. Nevertheless, temperatures lower than 50°C can be used, although, in this case, very high pressures would be required.

Next, the supercritical fluid loaded with lycopene is submitted to a depressurisation to separate the lycopene concentrate FOS. This stage is very important.

Due to the nature of the lycopene source itself, this will contain, in addition to the lycopene, some lipidic compounds, mainly triglycerides, that can be extracted by the supercritical fluid together with the lycopene. In general, these lipidic compounds are quite soluble in carbon dioxide (supercritical fluid) at moderate temperatures and pressures, for example, 20 MPa and 40-50°C, whereas the lycopene is insoluble in these conditions. These characteristics permit the concentration of lycopene in the concentrate to be manipulated because:
a) if the supercritical fluid loaded with lycopene is depressurised to low pressures, lower than 10 MPa, the lycopene precipitates and most of the lipidic components (mainly triglycerides), and a lycopene concentrate free of organic solvents is obtained that has been identified in this description as a "lycopene oleoresin FOS" and which is not part of the invention; this oleoresin (a natural product of vegetable origin comprised of a mixture of resins and essential oils that are obtained by submitting the plant to an extraction process) is a similar product to that obtained by extraction with organic solvents but with the advantage that products harmful to the health have not been used; and
b) if the supercritical fluid loaded with lycopene is depressurised to achieve relatively high pressures, equal to or higher than 10 MPa, preferentially, around 20 MPa, and at a temperature between 40°C and 60°C, the lycopene is not soluble and precipitates whereas most of the other components do not precipitate and leave the separator dissolved in the supercritical fluid, and therefore, in this case, a lycopene concentrate FOS is obtained that has been called in this description "extract rich in lycopene FOS", instead of an oleoresin since almost all the other components have been eliminated and this has a superior lycopene content. The extract of lycopene FOS obtained in this way, as well as being free of organic solvents, since organic solvents are not used for the lycopene extraction, is also substantially lipid free.

Owing to the use of supercritical fluid in the extraction process, at the end of this when the extracted product returns to atmospheric pressure, the fluid passes to the gaseous state and is completely eliminated without leaving any trace. Similarly, at the recommended working temperatures, the extract obtained does not undergo any structural modification and the lycopene obtained can be considered to be a completely natural lycopene.

The invention also provides a lycopene concentrate FOS obtained by following the procedure of the invention. Depending on the concentration of the lycopene FOS, which depends on the depressurisation conditions used, the concentrate can be an oleoresin rich in lycopene FOS which is not part of the invention or an extract rich in lycopene FOS.

In the sense it refers to in this description, the expression "rich in lycopene" signifies that the concentrate (oleoresin which is not part of the invention or extract) contains lycopene in a concentration higher than that present in the natural product from which it has been obtained (lycopene source). In general, the concentration of lycopene in the lycopene concentrate FOS is equal to or greater than 100 times, and preferentially equal to or greater than 500 times, the concentration of lycopene present in the natural products used as a lycopene source. As an example, the concentration of lycopene in an extract rich in lycopene obtained from industrial wastes of tomato processing, comprised of tomato skins, following the procedure of the invention was 37,120 ppm, whereas using fresh tomatoes as a starting material this contained 53 ppm.

In the sense used in the description, the expression "free of organic solvents" implies that the product completely lacks organic solvents since no organic solvent has been used in the procedure followed to obtain it. One of the essential aspects of this invention is precisely that the lycopene concentrate is free of organic solvents. The ability to be able to guarantee that no organic solvent has been used during the procedure followed to obtain the lycopene and to elaborate the different products is essential since one of the potential markets for the lycopene concentrates provided by this invention is for their use in nutraceutical products. As far as the inventors of this patent are aware, pure lycopene free of organic solvents did not previously exist, since in previously known procedures the dissolution of lycopene in organic solvents is a previous step to their purification.

In the sense intended in this description, the expression "extract rich in lycopene FOS" refers to a product that can be obtained by the procedure of the invention by depressurising at relatively high pressures, in which the lycopene FOS is the majority component. Therefore, the extract rich in lycopene FOS does not necessarily have to correspond to pure lycopene, although lycopene FOS must be the majority component. In one specific application, the extract rich in lycopene FOS has a lycopene FOS contents equal to or higher than 50% in weight, preferentially, equal to or higher than 70% in weight.

In another specific application, when the lycopene source is tomato or industrial wastes of the tomato processing industry comprised of tomato skins and seeds, the second most important component of the extract rich in lycopene FOS is usually β-carotene, another carotenoid with interesting nutritional properties. In this case, when tomato skin is used as a lycopene source, in which the ratio lycopene:β-carotene is 4.5:1 (depending on the variety of tomato), it is possible to obtain, by following the procedure of the invention, an extract rich in lycopene FOS with approximately 70% in weight of lycopene (depending on different variables of the procedure used to obtain the extract)and in which in the remaining 30% there is approximately 15% of β-carotene, i.e. this extract can contain around 85% of cartooned of great nutritional interest, whereas the remaining 15% would be comprised of numerous minority components.

In the sense intended in this description, the expression "oleoresin rich in lycopene FOS" refers to an oleoresin which is not part of the invention natural product of vegetable origin comprised of a mixture of the resin and essential oils that are obtained by submitting the plant to an extraction process) obtained via a procedure similar to that of the invention but by depressurising at relatively low pressures, with a lycopene content higher than that found in the natural product from which it is obtained, which is, also free of organic solvents. In one specific application, the concentration of lycopene in this oleoresin is equal to or greater than 100 times, and preferentially greater than 500 times, the concentration of lycopene present in the natural products. The concentration of lycopene FOS in this oleoresin can vary over a very wide range since it depends on the starting material used and in the conditions used to obtain it. In one specific application, when the starting material corresponds to tomato skins, an oleoresin that contains, as percentage weight relative to the total, 10% lycopene, 2.2% β-carotene, 16.2% of palmitic acid, 5.1% of stearic acid, 11.9% of oleic acid, 43.4% of linoleic acid, 7.7% of linolenic acid and other minority components.

The lycopene concentrate FOS provided by this invention has colorant and antioxidant properties, and easy absorption and can be used to elaborate products that contain these concentrates.

Therefore, the invention provides a composition, from hereon referred to as the composition of the invention, that includes this lycopene concentrate FOS in combination with an appropriate diluent. Additionally and optionally, the composition of the invention can contain one or more acceptable additives, for example antioxidants, emulsifying agents or mixtures of these. The composition of the invention can be a food, cosmetic, pharmaceutical or nutraceutical product.

The composition of the invention can be obtained by diluting the concentrate of the invention with a diluent to optionally, by adding one or two more appropriate additives, for example, antioxidants, emulsifiers and mixtures of these.

The composition of the invention can contain a variable amount of lycopene FOS, depending on the application for which it is to be used.

As a diluent, any substance can be used in which the lycopene is soluble and is permitted by the alimentary or pharmacopoeia regulations applicable in the country for which the product is destined, for example, fats, oils and mixtures of these. In one specific application, the diluent is comprised of one or more vegetable oils, for example, olive oil, walnut oil, sunflower oil, rapeseed oil etc. In one specific, and preferred application, this diluent is olive oil, preferentially virgin olive oil, since this is a natural product that can be obtained without requiring to use solvents and has important levels of tocopherols. The latter are compounds with antioxidant properties that have a synergic effect with the lycopene present in the composition of the invention. Moreover, several studies have demonstrated that olive oil has a preventive action against cardiovascular diseases.

As an antioxidant, any antioxidant permitted by the alimentary regulations of the country for which the product is destined can be used, for example ascorbic acid (vitamin C), tocopherols (vitamin E etc).

As an emulsifier, any emulsifier permitted by the alimentary regulations of the country where the product is destined can be used, for example, lecithin, monoglycerides etc.

In one specific application, it is provided a composition which is not part of the invention comprised of an oleoresin at 5% in lycopene FOS and a product of high nutritional value, for example, virgin olive oil. This composition can be consumed directly or used in products, for example, salad dressings.

The composition of the invention can be presented in any presentation form, liquid or solid, for example, encapsulated in soft gelatine capsules. These capsules are suitable for direct consumption by the consumer.

In one specific application of the encapsulated oleoresin which is not part of the invention this should be encapsulated under nitrogen, according to the usual techniques [Fauli and Trillo (1993)], in soft gelatine capsules that should be coloured in order to prevent any degradation effect with light. All the products obtained should be packaged under nitrogen and in opaque containers that keep out the light.

Although there is no unanimous agreement about the daily amount of lycopene recommended, this ranges from 5 to 10 mg, although studies using higher concentrations have shown no damaging effects.

An additional advantage of the products provided by this invention, both of the lycopene concentrate FOS and the compositions that comprise this FOS concentrate lies in the fact that, in contrast with natural lycopene sources, these favour absorption of lycopene in the intestinal tract. Indeed, in natural lycopene sources, for example, in the tomato, lycopene is occluded in the chromoplasts, which are cellular organelles surrounded by a wall, and this, to a certain extent, impedes absorption in the intestine. generates wastes containing lycopene, for example, in the tomato processing industry, since the residue obtained in this type of industry is an excellent source of lycopene. Concentrates of lycopene FOS provided by this invention, and the compositions they contain, are mainly destined for the nutraceutical products industry, resulting in an increased added value and, therefore, an important source of income for this industry.

The following examples illustrate the invention and should not be considered as limiting its scope of application.

### EXAMPLE 1

### Obtaining an extract rich in lycopene from an industrial waste made from tomato skins.

In this example, the procedure of obtaining an extract rich in lycopene from an industrial waste comprised of tomato skins is described. The tomato skins are dried until they have a humidity lower than 10%, ensuring that the product does not exceed a temperature of 50-60°C. Then, they are ground to achieve a particle size between 0.3 and 1.5 mm and the raw material is introduced into the extractor of a supercritical extraction apparatus and thermostated to a working temperature between 60°C and 80°C. After reaching this temperature a carbon dioxide current previously heated to the same temperature is passed through it and it is submitted to a working pressure between 30 and 70 MPa. The carbon dioxide, with the dissolved solids, is passed through the first separator, which is operating under preselected conditions, for example, 20 MPa and 40°C, conditions in which an extract with a majority lycopene component is obtained (>50%).

The carbon dioxide, together with the remaining solutes, can pass through some filters to eliminate these solutes and, after having been purified, can be sent again to the compressor or can pass to a second extractor that is maintained at a low pressure, between 1 and 2 MPa and a temperature between 0 and 20°C so that all the compounds still solubilised can precipitate and the carbon dioxide is then sent to the compressor.

In one specific application tomato skins with a particle size of 0.767 mm and a humidity of 6% were used. The extraction temperature was 80°C and the pressure 30 MPa. In the first separator the conditions 20 MPa and 40°C. An extract with a lycopene contents of 86% was obtained.

### EXAMPLE 2 (not part of the invention)

### Obtaining an oleoresin rich in lycopene from an industrial waste comprised of tomato skins.

The procedure of Example 1 was repeated but, in this case, the decompression was done in a single separator at low pressure, ranging from 1 to 2 MPa and a temperature between 0 and 20°C so that all the compounds still solubilised would precipitate, after which the carbon dioxide was sent to the compressor. An oleoresin with a purity of 3.712% was obtained i.e. 37,120 ppm.

### EXAMPLE 3 (not part of the invention)

### Soft oleoresin capsules

To obtain soft gelatine capsules containing oleoresin rich in lycopene FOS, first an oleoresin with a suitable concentration must be prepared. For example, an oleoresin is prepared at 0.60% in weight (6.0g lycopene/kg of oleoresin) by diluting an extract rich in lycopene or an oleoresin obtained according to Examples 1 or 2 with an appropriate amount of virgin olive oil (diluent).

As an example, if we start with an oleoresin at 10% we must add 15.67 kg of olive oil per kg of oleoresin at 10%, whereas if we start with an extract with a 60% lycopene content, we must dissolve 10 g of this extract per kg of olive oil. In both cases, the oleoresin at 0.6% obtained is homogenised by shaking under vacuum (for example at 100 rpm). At all times, the presence of sunlight should be avoided since lycopene is very photosensitive.

After homogenisation, the oleoresin is encapsulated by the usual methods, under nitrogen, in soft capsules prepared from caramel-coloured gelatine to obtain an encapsulated product in soft gelatine capsules. Each capsule is filled with 0.5g of oleoresin and, therefore, each capsule contains 3 mg of lycopene.

The invention permits a new encapsulated product to be obtained, of nutritional interest that has been prepared from natural products without using organic solvents in any stage of the procedure. Recommended intake of this product would be 3 capsules/day, that would supply a total of 9 mg.

Using the previously described product as a base, other products can be elaborated that also contain additional ingredients per capsule:

| | |
|---|---|
| Emulsifier | 1-15 parts in weight (mg) |
| Antioxidant | 1-15 parts in weight (mg) |
| Total contents per capsule | 500 parts in weight (mg) |

The state of the art contains numerous references that support the beneficial effects of lycopene supplements, optionally taken together with other products with a high nutritional value. As an example, the health benefits are reflected in the articles referred to in Background of the Invention. An important property of the products provided by this invention is that these have a better absorption than natural lycopene. Studies performed by Drs. W. Stahl and H. Sies, from the University of Dusseldorf, demonstrate that the presence of oil drastically increases the absorption of lycopene [Stahl & Sies (1996)].

### REFERENCES

Burton, G. W. (1989). "Antioxidant action of cartooned". J. Nutr. 119, 109-111.

Castro Gomez, F.J. (1999). "Estudio de la extraccion del licopeno procedente de los residuos industriales del tomate mediante el empleo de disolventes". Proyecto Fin de Carrera. Escuela de Ingenierias Industriales. Universidad de Extremadura.

De Felice, S.L. (1991). "The nutraceutical initiatives: A proposal for economic and regulatory reform". Ed. The Foundation for Innovation in Medicine.

Di Mascio, P.; Murphy, M.E. and Sies, H. (1989). "Lycopene as the most efficient biological carotenoid singlet oxigen quencher". Arch. Biochem. Biophys., 274, 532-538.

Di Mascio, P.; Murphy, M.E. and Sies, H. (1991). "Antioxidant defence systems: The role of cartooned, tocopherols and thiols". Am. J. Clin. Nutn., 53, 1945-2005.

Diplock, A. T. (1991). "Antioxidant nutrients and disease prevention: an overview". Am. J. Clin. Nutr. 53, 189S-193S.

Fauli and Trillo, C. (1993). "Capsulas de gelatina blandas", en Tratado de Farmacia Galenica, 1^{a} edicion, Luzan 5, S.A. de Ediciones, paginas 587-592.

Giovannucci, E. (1998). "Tomato intake and cancer risk: A review of the epidemiologic evidence". 3^{rd} Worldwide Congress of the Tomato Processing Industry. Pamplona, 25-28 de mayo 1998. pages 69-80.

Giovannucci, E.; Ascherio, A.; Rimm, E.B.; Stampfer, M.J.; Colditz, G.A.; Willett, W.C. (1995). "Intake of cartooned and retinol in relation to risk of prostate cancer". J. Natl. Cancer. Inst., 87, 1767-1776.

Rizvi, S.S.H., Daniels, J.A., Benado, A.L. and Zollweg, J.A., (1986). "Supercritical fluid extraction: operating principles and food applications". Food Technology July, 40(7), 57.

Stahl, W., & Sies H., Archives of Biochemistry and Biophysics, Vol. 336, No. 1 (1996).

## Claims

1. A procedure to obtain a lycopene concentrate free of organic solvents (FOS) that consists of:
a) placing in contact a source of lycopene with a supercritical fluid, under conditions that permit the solubilisation of lycopene in this supercritical fluid, and
b) separation of the lycopene concentrate FOS by depressurising the supercritical fluid loaded with lycopene obtained in stage a) ;
**characterised in** the supercritical fluid loaded with lycopene is depressurised to a pressure equal to or higher than 10 Mpa and a to a temperature between 40°C and 60°C.

2. Procedure according to claim 1, in which this source of lycopene ie a product that contains lycopene.

3. Procedure according to claim 1, in which this lycopene source corresponds to the industrial wastes from the tomato processing industry.

4. Procedure according to claim 1, in which this lycopene source corresponds to tomato skins, optionally together with tomato seeds.

5. Procedure according to claim 1 that corresponds to performing a drying and milling of the lycopene source before this is placed in contact with the supercritical fluid.

6. Procedure according to claim 5 in which this drying stage is performed until a product with a degree of humidity between 1, and 10% is obtained.

7. Procedure according to claim 5, in which this milling stage of the lycopene source is performed until a product with a particle size between 0.3 and 1.5 mm is obtained.

8. Procedure according to claim 1, in which this supercritical fluid is selected from among carbon dioxide, ethylene, ethane, chlorotrifluoromethane and propylene.

9. Procedure according to claim 8 in which this supercritical fluid is carbon dioxide.

10. Procedure according to claim 1, in which stage a) is carried out at a working pressure equal to or greater than 30 MPa.

11. Procedure according to claim 10, in which stage a) is carried out at a working pressure between 30 MPa and 70 MPa.

12. Procedure according to claim 1, in which stage a) is carried out at a temperature equal to or lower than 80°C.

13. Procedure according to claim 12 in which stage a) is carried out at a temperature between 50°C and 60°C.

## Patentansprüche

1. Verfahren zum Erhalt eines von organischen Lösungsmitteln freien (FOS) Lycopenkonzentrats, das aus folgenden Schritten besteht:
a) Inkontaktbringen einer Quelle von Lycopen mit einer überkritischen Flüssigkeit unter Bedingungen, die die Solubilisierung von Lycopen in dieser überkritischen Flüssigkeit erlauben, und
b) Trennung des FOS-Lycopenkonzentrats durch Druckablassen der in Stufe a) erhaltenen, mit Lycopen beladenen überkritischen Flüssigkeit;
**dadurch gekennzeichnet, dass** die mit Lycopen beladene überkritische Flüssigkeit auf einen Druck von gleich oder mehr als 10 MPa und auf eine Temperatur zwischen 40 und 60°C abgelassen wird.

2. Verfahren gemäß Anspruch 1, worin die Quelle von Lycopen ein Erzeugnis ist, das Lycopen enthält.

3. Verfahren gemäß Anspruch 1, worin die Lycopenquelle den gewerblichen Abfällen aus der tomatenverarbeitenden Industrie entspricht.

4. Verfahren gemäß Anspruch 1, worin die Lycopenquelle Tomatenhaut entspricht, ggf. zusammen mit Tomatensamen.

5. Verfahren gemäß Anspruch 1, das dem Durchführen von Trocknen und Mahlen der Lycopenquelle entspricht, bevor sie mit der überkritischen Flüssigkeit in Kontakt gebracht wird.

6. Verfahren gemäß Anspruch 5, worin die Trocknungsstufe durchgeführt wird, bis ein Erzeugnis mit einem Feuchtigkeitsgrad zwischen 1 und 10% erhalten wird.

7. Verfahren gemäß Anspruch 5, worin die Mahlstufe der Lycopenquelle durchgeführt wird, bis ein Erzeugnis mit einer Teilchengröße zwischen 0,3 und 1,5 mm erhalten wird.

8. Verfahren gemäß Anspruch 1, worin die überkritische Flüssigkeit aus Kohlendioxid, Ethylen, Ethan, Chlortrifluormethan und Propylen ausgewählt ist.

9. Verfahren gemäß Anspruch 8, worin die überkritische Flüssigkeit Kohlendioxid ist.

10. Verfahren gemäß Anspruch 1, worin Stufe a) bei einem Arbeitsdruck von gleich oder mehr als 30 MPa durchgeführt wird.

11. Verfahren gemäß Anspruch 10, worin Stufe a) bei einem Arbeitsdruck zwischen 30 und 70 MPa durchgeführt wird.

12. Verfahren gemäß Anspruch 1, worin Stufe a) bei einer Temperatur von gleich oder weniger als 80°C durchgeführt wird.

13. Verfahren gemäß Anspruch 12, worin Stufe a) bei einer Temperatur zwischen 50 und 80°C durchgeführt wird.

## Revendications

1. Un procédé d'obtention d'un concentrat de lycopène exempt de solvants organique (FOS), qui consiste:
a) à placer une source de lycopène au contact d'un fluide super-critique, dans des conditions qui permettent la solubilisation du lycopène dans ce fluide super-critique, et
b) à séparer par dépressurisation, du concentrat de lycopène POS le fluide super-critique chargé de lycopène,
ce procédé étant **caractérisé en ce que** le fluide super-critique chargé de lycopène est dépressurisé à une pression égale ou supérieure à 10 Mpa et à une température comprise entre 40°C et 60°°C.

2. Un procédé selon la revendication 1, dans lequel la source de lycopène est un produit qui contient du lycopène.

3. Un procédé selon la revendication 1, dans lequel cette source de lycopène est constituée par les déchets industriels qui sont produits dans l'industrie du traitement des tomates.

4. Un procédé selon la revendication 1, dans lequel dans lequel cette source de lycopène est constituée par des peaux de tomates, le cas échéant additionnées des graines de tomates.

5. Un procédé selon la revendication 1, dans lequel on effectue le séchage et le broyage de la source de lycopène préalablement à sa mise en contact avec le fluide super-critique.

6. Un procédé selon la revendication 5, dans lequel cette étape de séchage est poursuivie jusqu'à l'obtention d'un taux d'humidité soit compris entre 1 et 10%.

7. Un procédé selon la revendication 5, dans lequel l'étape de broyage de la source de lycopène est poursuivie jusqu'à l'obtention d'un produit dont la dimension particulière soit comprise entre 0,3 et 1,5 mm.

8. Un procédé selon la revendication 1, dans lequel ce fluide super-critique est choisi dans le groupe comprenant le dioxyde de carbone, l'éthylène, l'éthane, le chlorotrifluorométhane et le propylène.

9. Un procédé selon la revendication 8, dans lequel ce fluide super-critique est le dioxyde de carbone.

10. Un procédé selon la revendication 1, dans lequel l'étape a) est mise en oeuvre sous une pression opératoire égale ou supérieure à 30Mpa.

11. Un procédé selon la revendication 1, dans lequel l'étape a) est mise en oeuvre sous une pression opératoire comprise entre 30Mpa et 70Mpa.

12. Un procédé selon la revendication 1, dans lequel l'étape a) est mise en oeuvre à une température égale ou supérieure à 80°C.

13. Un procédé selon la revendication 1, dans lequel l'étape a) est mise en oeuvre à une température comprise entre 50°C et 80°C.
